# EUROPEAN PATENT APPLICATION

(11) **EP 1 757 578 A1**
(43) Date of publication of application: **28.02.2007**
(21) Application number: 05730649.0
(22) Date of filing: 15.04.2005
(51) Int. Cl.: C07C 67/54, C07C 69/54, C07C 69/653

(54) **PROCESS FOR PRODUCING FLUORINATED ACRYLIC ESTER**

(30) Priority: 26.04.2004 JP 2004129715
(71) Applicant: DAIKIN INDUSTRIES, LTD., Osaka-shi, Osaka 530-8323 (JP)
(72) Inventor: HIRASAKA, Takeomi, DAIKIN INDUSTRIES, LTD., Settsu-shi, Osaka 5668585 (JP); TANAKA, Yoshinori, DAIKIN INDUSTREIS, LTD., Settsu-shi, Osaka 5668585 (JP); FUNAKOSHI, Yoshio c/o Yodogawa Plant of DAIKIN, Settsu-shi, Osaka 5668585 (JP); YAMAMOTO, Osamu, DAIKIN INDUSTRIES, LTD., Ibaraki 3140255 (JP)
(74) Representative: HOFFMANN EITLE
(86) International application number: PCT/JP2005/007330
(87) International publication number: WO 2005/102984

(57) **Abstract**

A mixture of fluorine-containing acrylic esters represented by CF₃(CF₂)ₙCH₂CH₂OCOCR¹=CH₂ wherein R¹ is a hydrogen atom, a methyl group or a halogen atom and "n" is an integer of at least zero is subjected to distillation under such conditions that the esters are not polymerized, so as to give a mixture of the esters with a less content of impurities (that is, olefins represented by CF₃(CF₂)ₙCH=CH₂ and alcohols represented by CF₃(CF₂)ₙCH₂CH₂OH) at a high yield.

## Description

### Technical Field

The present invention is related to a method for producing a mixture of fluorine-containing acrylic esters which includes less impurities.

### Background Art

A fluorine-containing acrylic ester represented by a general formula (1):

CF₃(CF₂)ₙCH₂CH₂OCOCR¹=CH₂ (1)

wherein R¹ is a hydrogen atom, a methyl group or a halogen atom and "n" is an integer of at least zero is used as a monomer in production of a fluorine-containing acrylate-based polymer that may be an active component for a water-and oil-repellent. Various methods for producing this monomer have been proposed.

For example, Japanese Patent Kokoku Publication No. S39-18112(1964) and Japanese Patent Kokoku Publication No. S48-30611(1973) disclose a method for reacting a fluorine-containing alkyl halide with an alkali metal salt of a carboxylic acid in a specific solvent. Japanese Patent Kokoku Publication Nos. H4-16451(1992), H4-16452(1992) and S61-57813(1986) disclose a method for producing the fluorine-containing ester and mention that the resultant fluorine-containing ester may be converted to an acrylic ester. Further, Japanese Patent Kokai (Laid-Open) Publication No. H9-59215(1997) discloses a method for producing the fluorine-containing acrylic ester represented by the above formula (1) is produced by reacting the fluorine-containing alkyl halide with a specific betaine followed by an alkaline treatment.

Another method for producing the fluorine-containing acrylic ester is known wherein the fluoroalkyl halide is converted to the fluorine-containing alkyl alcohol that is then converted to the fluorine-containing acrylic ester. For example, in Japanese Patent Kokai (Laid-Open) Publication No. S59-181239(1984), a method for producing the fluorine-containing acrylic ester is disclosed wherein the fluorine-containing alkyl alcohol and acrylic acid or methacrylic acid are reacted under the presence of a concentrated sulfuric acid or a fuming sulfuric acid. In Patent Kokai Publication (Laid-Open) No. H2-295948(1990), a method for reacting the fluorine-containing alkyl alcohol and methacrylic acid under the presence of phosphoric anhydride is disclosed. USP No. 3,719,698 discloses a method for producing the fluorine-containing acrylic ester wherein the fluorine-containing alkyl alcohol is added to a compound that is obtained by reacting acrylic acid or methacrylic acid with a trifluoroacetic anhydride. Japanese Patent Kokai (Laid-Open) Publication Nos. S59-117503(1984), S59-117504(1984) and H3-163044(1991) disclose a method for producing the fluorine-containing acrylic ester wherein the acrylic acid halide or the methacrylic acid halide is reacted with the fluorine-containing alcohol in an aqueous solution of an alkali metal hydroxide.

In any methods, a fluoroalkyl iodide generally represented by R_{f}I (R_{f} is a fluoroalkyl group) is used as a starting material. In the method disclosed in Patent Kokoku Publication Nos. S39-18112(1964) and S48-30611(1973), an ethylene adduct of this iodide is used. In the method using the fluoroalkyl alcohol as described in Japanese Patent Kokoku Publication No. S61-57813(1986), this iodide is used as a material for the alcohol. R_{f}I is, for example, a telomer produced by a telomerization reaction.

### Disclosure of Invention

### (Problems to be Solved by Invention)

It is known that when the monomer represented by the formula (1) is polymerized, either or both of an olefin represented by the following formula (2) and an alcohol represented by the following formula (3) are contained in addition to a polymer in the resultant. These compounds do not function as effective components for the water- and oil-repellent.

CF₃(CF₂)ₙCH=CH₂ (2)

CF₃(CF₂)ₙCH₂CH₂OH (3)

Further, it is known that the monomer constituting the polymer for the water- and oil-repellent is preferably the monomer with "n" of at least 7 in the above formula (1). When the polymerization is carried out using a mixture of the monomers with "n" of at least 7, compounds represented by the formulas (2) and (3) of n=7, that is, either or both of C₈F₁₇CH=CH₂ and C₈F₁₇CH₂CH₂OH are contained in the polymer. Of these compounds, C₈F₁₇CH₂CH₂OH may be oxidized to give C₇F₁₅COOH (perfluoro octanoic acid: abbreviation PFOA). Recent research results for example, EPA report "PRELIMINARY RISK ASSESSMENT OF THE DEVELOPMENTAL TOXICITY ASSOCIATED WITH EXPOSURE TO PERFLUOROOCTANOIC ACID AND ITS SALTS" (http://www.epa.gov/opptintr/pfoa/pfoara.pdf) point out that perfluorooctanoic acid might be a burden to the environment. For this reason, the EPA (Environmental Protection Agency of US) announced, on April 14, 2003, that they would strengthen the scientific research as to PFOA.

The compounds represented by the formulas (2) and (3) are preferably removed from the polymer from the viewpoint of providing a product of high quality as described above, and thereby it may be possible to avoid the inclusion of PFOA in the final product. However, a specific method for removing these compounds has not been indicated. Consequently, the inventors have studied a possibility of removing these compounds by distillation. As a result, the inventors have made the present invention. Further, the inventors found a new problem that the distillation cannot be effectively conducted due to deterioration of an inhibitor of polymerization when the distillation is carried out with the inhibitor added so as to suppress the polymerization of the monomer represented by the formula (1). The inventors have made, as a result of study, the present invention in order to solve this problem.

### (Means to Solve the Problem)

In other words, the present invention provides a method for producing a mixture of fluorine-containing acrylic esters each of which esters is represented by the formula (1) :

CF₃(CF₂)ₙCH₂CH₂OCOCR¹=CH₂ (1)

wherein R¹ is a hydrogen atom, a methyl group or a halogen atom and "n" is an integer of at least zero, which includes:
(a) a step of obtaining a mixture containing a mixture of fluorine-containing acrylic esters each of which esters is represented by the above formula (1): and
(b) a step of reducing, in the mixture obtained in the step (a), a proportion of a mixture of fluorine-containing olefins each of which is represented by a formula (2) and/or a mixture of fluoroalkyl alcohols each of which is represented by a formula (3) by subjecting the mixture obtained in the step (a) to distillation:

   CF₃(CF₂)ₙCH=CH₂ (2)

   wherein "n" is an integer of at least 0,

   CF₃(CF₂)ₙCH₂CH₂OH (3)

   wherein "n" is an integer of at least 0,
   wherein the distillation is carried out using a distillation column equipped with a packing and/or an internal structure made of a material which does not contain a metal having a standard electrode potential of - 0.3V or less and introducing oxygen into the distillation column..

Herein, the "mixture of fluorine-containing acrylic esters" refers to a mixture containing two or more esters which have different "n" values in the formula (1). The fluorine-containing acrylic ester is generally obtained as such a mixture, but is can be obtained as a compound wherein "n" value is a particular single numeral. However, it seems unlikely that no other esters with other "n" values are contained in the compound when the compound with "n" of the particular single numeral is obtained. For this reason, the term "mixture" is used herein. Similarly, the term "mixture" as to a compound which includes, in its chemical formula, a letter such as "n" or the like corresponding to a polymerization degree is used herein in the sense that it includes a plurality of compounds with different "n" values. Further, in this specification, a symbol "≥" may be used to generically refer to compounds with "n" of "k" or more (at least "k") and a symbol "≤" may be used to generically refer to compounds with "n" of "k" or less (or at most "k"). Furthermore, a mixture which does not contain a compound with "n" of zero (specifically, the fluorine-containing acrylic ester or the fluoroalkyl alcohol) is identified as a mixture with "n" of one or more and such a mixture can be employed or produced in the present invention.

This production method is characterized in that the distillation is carried out so that the mixing proportion of the compound represented by the formula (2) and/or the compound represented by the formula (3) is reduced after the mixture of the fluorine-containing esters represented by the formula (1) is obtained. The distillation makes it possible to give the mixture wherein the proportion of the esters represented by the formula (1) is higher. The distillation is an effective method for removing the compounds represented by the formulas (2) and (3) and suitable for industrial mass production. The fluorine-containing acrylic ester is prevented form being polymerized during the distillation by forming the packing and/or the internal structure in the distillation column of the material which does not contain a metal of a low standard electrode potential and introducing oxygen into the distillation column.

In the production method of the present invention, the step (a) corresponds to a step of carrying out a conventional method for producing the mixture of the fluorine-containing acrylic esters. Specifically, the step (a) is carried out according to, for example, any of the methods described in the references cited in the above.

The present invention also provides a method for producing a polymer by polymerizing the mixture of the fluorine-containing acrylic esters produced according to the production method of the present invention. Since the starting materials (monomers) with less impurities are used in the method for producing the polymer of the present invention, the resultant polymer also contains less impurities.

Further, when only the step (b) is carried out, the present invention provides a purification method for increasing a purity of the fluorine-containing acrylic esters.

### (Effect of Invention)

The production method of the present invention makes it possible to obtain the mixture of the fluorine-containing acrylic esters which contains less impurities. The ester mixture with less impurities improves the quality of the final product (for example, the water- and oil-repellent) which is obtained by polymerizing the esters. Further, the production of PFOA resulting from the impurities can be reduced.

### Embodiments for Carrying Out the Invention

Each step is described below to explain the method of the present invention for producing the mixture of the fluorine-containing acrylic esters.

The step (a) is carried out according to any method for synthesizing the fluorine-containing acrylic esters represented by the formula (1), as described above. Specifically, for example:
(a-1) a method wherein a mixture of fluoroalkyl iodides each of which is represented by a formula:

   CF₃(CF₂)ₙI

   wherein "n" is an integer of at least 0, is produced by a telomerization reaction in which CFₘ(CF₂)₂ₘ₊₁I wherein "m" is any one integer of 1 or more is telogen and tetrafluoroethylene is taxogen, and ethylene is added to the mixture to give a mixture of ethylene adducts each of which is represented by CF₃(CF₂)ₙCH₂CH₂I, and then the mixture of the ethylene adducts is reacted with an acrylic acid compound, or
(a-2) a method wherein a mixture of fluoroalkyl iodides each of which is represented by a formula:

   CF₃(CF₂)ₙI

   wherein "n" is an integer of at least 0, is produced by a telomerization reaction wherein CFₘ(CF₂)₂ₘ₊₁I wherein "m" is any one integer of 1 or more is telogen and tetrafluoroethylene is taxogen, and ethylene is added to the mixture to give a mixture of ethylene adducts each of which is represented by CF₃(CF₂)ₙCH₂CH₂I, and the adduct mixture is converted to alcohol to give a mixture of CF₃(CF₂)ₙCH₂CH₂OH and then this mixture is reacted with an acrylic acid compound
   is carried out to give the mixture of the esters represented by the formula (1).

It is known that the polymer useful as the active component for the water- and oil-repellent is the polymer with "n" of seven or more in the formula (1). Therefore, the step (a) is preferably carried out so that the resultant mixture contains the esters of n≥7 in a large amount. For example, when the step (a) is carried out according to the method of (a-1), it is preferable that an operation (specifically, distillation) for obtaining a mixture wherein the fluoroalkyl iodides of n≥7 are contained in a large amount is carried out before the ethylene addition and then the subsequent ethylene addition and the like is carried out. Alternatively, it is preferable that the mixture after the ethylene addition is subjected to distillation so as to obtain another mixture containing the ethylene adducts of n≥7 in a large amount and then the another mixture is reacted with the acrylic acid compound. Similarly, in the case where the step (a) is carried out according to the method (a-2), it is preferable that the mixture obtained by adding ethylene to the fluoroalkyl iodides is subjected to distillation so as to obtain another mixture containing the ethylene adducts of n≥7 in a large amount and then the another mixture is converted to alcohol. Alternatively, it is preferable that the alcohol mixture is subjected to distillation so as to obtain another alcohol mixture containing the alcohols of n≥7 in a large amount, and then the another mixture is reacted with the acrylic acid compound.

The step (b) is carried out as the distillation step. When the distillation is carried out industrially, it is carried out as rectification. A preferable distillation method in the production method of the present invention is described below.

The distillation of the step (b) is preferably carried out adding an inhibitor of polymerization to the mixture obtained in the step (a) in order to prevent the fluorine-containing acrylic esters from being polymerized during the distillation. The inhibitors of polymerization include, for example, hydroquinone or hydroquinone monomethylether, but are not limited thereto.

In the step (b), particularly in the case where the inhibitor of polymerization is carried out, the distillation is preferably conducted using a distillation column provided with a packing and/or an inner structure made of a material which does not contains a metal having a standard electrode potential of -0.3V or less. Herein, the "internal structure" means a member disposed inside the distillation column, and it is, for example, a porous plate, a baffle plate, a shuttering and a downcomer. The inventors found, as a result of various examinations, that the inhibitor of polymerization may not show effect of preventing the polymerization when it is heated to a high temperature. The cause of this was extensively investigated, and it was found that the monomers represented by the formula (1) are unstable at the high temperature and polymerized beyond a range within which the effect of the inhibitor is obtained. For this reason, the deterioration of the inhibitor is preferably avoided as far as possible. However, the inventors further found that when a metal having a low standard electrode potential, such as iron, exists inside the distillation column, the inhibitor is deteriorated by being affected by the metal, resulting in tendency of production of impurities, and that the polymerization tends to occur because the effective amount of the inhibitor is decreased due to the deterioration. Based on this knowledge, the present invention requires that the packing and/or the internal structure inside the distillation column is made of a material which does not accelerate the deterioration of the inhibitor. Such a material is a material that is free from the metal having a standard electrode potential of -0.3V or less as described above, and specifically, a resin, a metal oxide, a metal having a standard electrode potential of larger than -0.3V, or a mixture thereof. When the packing and/or the internal structure is made of, for example, a mixture of a resin and a metal, the metal needs to be one having the standard potential of larger than -0.3V irrespective of a mixing ratio of the metal. The resins suitable for constituting the packing and/or the inner structure include, for example, poly(perfluorooctylethyl acrylate), polytetrafluoroethylene, hexafluoroethylene-hexafluoropropylene copolymer resin, polyphenylene sulfide, polyetheretherketone, and polyether nitrile. The metal oxide suitable for constituting the packing and/or the inner structure is, for example, alumina or the like. The metals suitable for forming the packing and/or the inner structure include, for example, copper and nickel. Alternatively, the packing and/or the inner structure may be formed of glass. The packing is packed in a form of particles into the distillation column. The packing may be particles formed of a mixture of two or more materials. Alternatively, the packing may be packed into the distillation column by mixing two or more types of particles which are made of resins different from each other, or by mixing resin particles and metal particles.

Further, a lining made of the material which does not contain the metal having the standard electrode potential of -0.3V or less is preferably used in the distillation column in order that the deterioration of the inhibitor of polymerization is suppressed. In the case where not only the packing and the internal structure but also the surface of the inner wall of the distillation column is a surface of the material not containing the metal having the standard electrode potential of -0.3V or less, the inhibitor of polymerization is more effectively prevented from being deteriorated, and therefore the polymerization of the fluorine-containing acrylic esters is more effectively prevented. The resins and metals suitable for forming the lining are as described in conjunction with the packing and so on. The lining may be made of a mixture of two or more resins, or a laminate of two or more layers. The lining may be made of glass.

Further, particularly in the case where the inhibitor of polymerization is used in the step (b), the distillation is preferably carried out introducing oxygen or a gas containing oxygen (for example, air) into the distillation column. The oxygen is preferably used because the oxygen itself serves as the inhibitor of polymerization and higher effect of inhibiting polymerization is achieved by synergistic effect of oxygen and the inhibitor of polymerization such as hydroquinone and hydroquinone monomethylether. In other words, the introduction of oxygen makes it possible to reduce the added amount of the polymerization inhibitor and therefore the amount of the impurities due to the polymerization inhibitor can be reduced in the resultant ester mixture. Oxygen or the gas containing oxygen is preferably introduced so that an oxygen concentration in the distillation column to the total moles of the gas-phase components in the distillation column is from 0.1 mol% to 10 mol%. When the gas containing oxygen is introduced in the distillation column, the introduced amount of the gas is adjusted depending on, for example, the proportion occupied by oxygen in gas.

The distillation in the step (b) is carried out for reducing the proportion of the compounds represented by the formula (2) and/or the compounds represented by the formula (3) contained in the mixture obtained in the step (a). Here, in the case where only the compounds represented by the formula (2) are contained as the impurities in the mixture obtained in the step (a), the distillation is carried out merely for removing the compounds. In the case where only the compounds represented by the formula (3) are contained as the impurities in the mixture obtained in the step (a), the distillation is carried out merely for removing the compounds. As a matter of course, both of the compounds represented by the formulas (2) and (3) may be contained in the mixture obtained in the step (a). The present invention may be applied to any of these cases, and the term "and/or" is used in that sense.

There is, as a conventional method for producing the fluorine-containing acrylic esters which is carried out as the step (a), a method wherein C₂F₅(CF₂CF₂)ₓI wherein "x" is an integer of 1 or more is used as a starting material, which is obtained by a telomerization reaction in which C₂F₅I is telogen and tetrafluoroethylene is a taxogen. The ester produced by using this fluoroalkyl iodide is represented by a formula (10):

C₂F₅(CF₂CF₂)ₓOCOCR¹=CH₂ (10)

wherein R¹ is a hydrogen atom, a methyl group or a halogen atom and "x" is an integer of at least one. The esters of n≥7 in the formula (1) correspond to the esters of x≥3 in the formula (10). It is preferable that, in the present invention, the step (a) is carried out so that a mixture is obtained wherein the sum of the fluorine-containing acrylic esters of x=3 and x=4 in the formula (10) (that is, n=7 and n=9 in the formula (1)) contained is 80 mol% or more; and the distillation of this mixture in the step (b) is carried out employing such a condition that the mixing ratio of the fluorine-containing olefins represented by the formula (2) with "n" of at most 9 is substantially 0 mol% and the mixing ratio of the fluorine-containing olefins with "n" of at least 10 is from 0 mol% to 0.05 mol% to the total moles of the fluorine-containing compounds obtained as a distillate. Alternatively, it is preferable that, in the present invention, the step (a) is carried out so that a mixture is obtained wherein the sum of the fluorine-containing acrylic esters of x=3 and x=4 in the formula (10) (that is, n=7 and n=9 in the formula (1)) contained is 80 mol% or more and the distillation of this mixture in the step (b) is carried out in such a condition that the mixing ratio of the fluorine-containing alcohols represented by the formula (3) with "n" of at most 8 is substantially 0 mol%, the mixing ratio of the fluorine-containing alcohols with "n" of 9 is from 0 mol% to 0.05 mol% and the mixing ratio of the fluorine-containing alcohols with "n" of at least 10 is from 0 mol% to 0.1 mol% to the total moles of the fluorine-containing compounds obtained as a distillate. The esters of x=3 and x=4 in the formula (10) can be obtained by, for example, subjecting the mixture of fluoroalkyl iodides to the distillation before the ethylene addition step, as described above. Herein, it should be noted that the expression that a component is "substantially 0 mol%" means that the component cannot be detected by usual gas chromatography and the mixture may contain the component in a minute amount within that range.

As described above, it is possible to obtain the monomer mixture with less impurities which may form the polymer useful as an active component for the water- and oil-repellent by carrying out the steps (a) and (b) so that the esters of n=7 and 9 are contained in a large amount. When the steps (a) and (b) are carried out as described above, the distillation in the step (b) is specifically carried out with the theoretical plate number of from 10 to 35, at a bottom temperature of from 60°C to 160°C and a pressure in the column of from 0.5kPa to 5kPa.

In the above, the method for producing the mixture containing the esters of n=7 and 9 in a large amount is exemplified as a preferable embodiment. The method of the present invention can be applied irrespective of "n" values. For example, the production method of the present invention may be applied to the case where only the ester of n=7 is intended to be produced, or the case where only the ester of n≤6 is intended to be produced. However, the problems due to the polymerization of the esters during the distillation and the deterioration of the inhibitor of polymerization significantly arise, as the "n" value of the component to be obtained in a large amount as the main component is larger (that is, the bottom temperature during the distillation in the step (b) is higher). Therefore, it is preferable to make the packing and/or the internal structure of the resin or the like in the distillation column used for the distillation in the step (b) and to introduce oxygen into the distillation column during the distillation in the step (b), in the case where the components with large "n" values, particularly n≥7 are intended to be obtained in a large amount.

The step (b) may be realized as a method for increasing the purity of the mixture of the fluorine-containing acrylic esters. In other words, the step (b) may be carried out as a method for purifying the fluorine-containing acrylic esters in the form of an independent method.

A fluorine-containing acrylate-based polymer can be obtained by subjecting the mixture obtained in the step (b) to the polymerization step. The polymerization may be carried out employing any polymerization condition which is conventionally employed. The resultant polymer has excellent quality since it contains less impurities. The resultant polymer is useful as the water- and oil-repellent for treating a surface of a substrate, such as textile products, stone material, a filter (for example, an electrostatic filter), a dust-protective mask, a fuel battery, glass, paper, wood, leather, fur skin, asbestos, brick, cement, metal and oxide, ceramic material, and plastics. Further, the resultant polymer is useful as a water- and oil-repellent and antifouling finish for carpeting.

### Examples

### (Example 1)

A mixture of ethylene adducts of fluoroalkyl iodides each of which adduct was represented by CF₃(CF₂)ₙCH₂CH₂I was obtained as a mixture containing the adducts of n≤6 in an amount of 5 mol%, the adduct of n=7 in an amount of 76 mol%, the adduct of n=8 in an amount of 0 mol%, the adduct of n=9 in an amount of 17 mol% and the adduct of n≥10 in an amount of 2 mol%. Next this mixture was reacted with an acrylic acid compound to give a mixture containing the fluorine-containing acrylic esters represented by the formula (1). Specifically, 1576g (2.67 mol) of the ethylene adducts of the fluoroalkyl iodides, 320g (2.90 mol) of potassium acrylate, 680mL of tert-butyl alcohol, and 1.8g of hydroquinone and 0.32g of hydroquinone monomethylether as inhibitors of polymerization were firstly charged into an autoclave with a volume of 3L and heated to 180°C to 190°C and reacted for 6 hours. After the reaction, a reaction mixture was cooled. Next, KI as a byproduct was removed by filtration. Thereafter, a filtrate was subjected to distillation to remove tert-butyl alcohol and then 1288g of a reaction mixture having a composition shown in Table 1 was obtained. 1000g of this reaction mixture was measured and charged into a still and then subjected to distillation for 2 hours at a pressure in a rectifier of 0.9kPa and a still temperature of 160°C, introducing air into the still at a flow rate of 20 ml/min such that the oxygen concentration in the distillation column was 1 mol% relative to the total moles of gas-phase components in the distillation column. Further, the continuous distillation was carried out using an oldershaw-type distillation column with 20 plates which was made of glass as a whole (including the internal structure). As a result of the distillation, 886g of a mixture after distillation having a composition as shown in Table 1 was obtained as a distillate liquid (a recovery rate based on the fluorine-containing acrylic esters was 97%). In Table 1, the compositions of the reaction mixture and the mixture after distillation were determined by gas chromatography.

**Table 1**

| Component | Reaction mixture (mol%) | Mixture after distillation (mol%) |
|---|---|---|
| C₆F₁₃CH=CH₂ | 0.91 | ND |
| C₈F₁₇CH=CH₂ | 13.65 | ND |
| C₁₀F₂₁CH=CH₂ | 3.09 | ND |
| C₁₂F₂₅CH=CH₂ | 0.34 | 0.01 |
| C₆F₁₃CH₂CH₂OH | 0.11 | ND |
| C₈F₁₇CH₂CH₂OH | 1.57 | ND |
| C₁₀F₂₁CH₂CH₂OH | 0.35 | 0.04 |
| C₁₂F₂₅CH₂CH₂OH | 0.04 | 0.05 |
| C₆F₁₃CH₂CH₂OCOCH=CH₂ | 4.09 | 5.1 |
| C₈F₁₇CH₂CH₂OCOCH=CH₂ | 60.73 | 75.93 |
| C₁₀F₂₁CH₂CH₂OCOCH=CH₂ | 13.54 | 16.92 |
| C₁₂F₂₅CH₂CH₂OCOCH=CH₂ | 1.58 | 1.96 |

As shown in Table 1, of the impurities contained in the reaction mixture, particularly C₈F₁₇CH=CH₂, C₁₀F₂₁CH=CH₂, C₈F₁₇CH₂CH₂OH and C₁₀F₂₁CH₂CH₂OH were able to be removed considerably by the distillation. As a result, the highly-pure mixture of the fluorine-containing acrylic esters was obtained, in which the contents of the fluorine-containing acrylic esters of n=7 and n=9 contained were large. Specifically, the mixture contained, as the impurities, C₁₀F₂₁CH₂CH₂OH (n=9) in an amount of 0.04 mol%, C₁₂F₂₅CH=CH₂ (n=11) in an amount of 0.01 mol% and C₁₂F₂₅CH₂CH₂OH (n=11) in an amount of 0.05 mol%.

### (Example 2)

The reaction mixture having a composition shown in Table 2 was obtained in the same manner as that in Example 1 except that a mixture of the ethylene adducts of fluoroalkyl iodides containing the adduct of n=7 in an amount of 80 mol% and the adduct of n=9 in an amount of 20 mol% was used. The mixture of the ethylene adducts containing ones of n=7 and n=9 was obtained by carrying out, before the ethylene addition, the rectification of the mixture of the fluoroalkyl iodides of n≥1 (which, however, does not substantially contain ones with "n" of an even number), in which distillation the initial distillate and the final distillate were cut in a large amount so that the fluoroalkyl iodides of n≤6 and n≥10 are not contained in the mixture that was then subjected to ethylene addition. 500g of this reaction mixture was measured and charged into the still with 22g of hydroquinone added, and then a continuous distillation was carried out at a pressure of the distillation column of 0.9kPa, a still temperature of 160°C, introducing air into the still at a flow rate of 20ml/min such that the oxygen concentration in the distillation column was 1 mol% to the total moles of the gas-phase components in the distillation column. The continuous distillation was carried out for 10 hours providing the reaction mixture to the still at a rate of 80g/hr so that the total weight of the reaction mixture to be subjected to distillation was 1000g. Further, the distillation column was the same as that used in Example 1. As a result, 908g of the mixture after distillation having a composition as shown in Table 2 was obtained (a recovery rate based on the fluorine-containing acrylic esters was 98%) was obtained. In Table 2, the compositions of the reaction mixture and the mixture after distillation were determined by gas chromatography.

**Table 2**

| Component | Reaction mixture (mol%) | Mixture after distillation (mol%) |
|---|---|---|
| C₈F₁₇CH=CH₂ | 13.54 | ND |
| C₁₀F₂₁CH=CH₂ | 3.41 | ND |
| C₈F₁₇CH₂CH₂OH | 0.88 | ND |
| C₁₀F₂₁CH₂CH₂OH | 0.18 | 0.03 |
| C₈F₁₇CH₂CH₂OCOCH=CH₂ | 65.58 | 80.31 |
| C₁₀F₂₁CH₂CH₂OCOCH=CH₂ | 16.41 | 19.66 |

As shown in Table 2, the highly-pure mixture of the fluorine-containing acrylic esters with less impurities was obtained in Example 2, similarly to Example 1. Specifically, only C₁₀F₂₁CH₂CH₂OH (n=9) was contained in an amount of 0.03 mol%.

### (Example 3)

A mixture containing the fluorine-containing acrylic esters represented by the formula (1) was obtained by obtaining a mixture of the ethylene adducts of the fluoroalkyl iodides, which adducts were represented by C₈F₁₇CH₂CH₂I (n=7) and then reacting this mixture with the acrylic acid compound. Specifically, 1533g (2.67 mol) of the ethylene adducts of fluoroiodides, 320g (2.90 mol) of potassium acrylate, 680mL of tert-butyl alcohol, and 1.8g of hydroquinone and 0.32g of hydroquinone monomethylether as inhibitors of polymerization were charged into an autoclave with a volume of 3L and heated to 180°C to 190°C and reacted for 6 hours. After the reaction, a reaction mixture was cooled. Next, KI as a byproduct was removed by filtration. Thereafter, a filtrate was subjected to distillation to remove tert-butyl alcohol and then 887g of a reaction mixture having a composition shown in Table 1 was obtained. 500g of this reaction mixture was measure and charged into a still and subjected to distillation for 6 hours at a pressure in a rectifier of 0.9kPa and a still temperature of 160°C, introducing nitrogen into the still at a flow rate of 20 ml/min such that the oxygen concentration was 0 mol% relative to the total moles of gas-phase components in the distillation column. Further, the distillation column used in this example was the same as that used in Example 1. As a result of distillation, 272g of C₈F₁₇CH₂CH₂OCOCH=CH₂ having a composition as shown in Table 1 was obtained as a distillate a distillate liquid (a recovery rate based on the fluorine-containing acrylic esters was 59%). In Table 1, the compositions of the reaction mixture and the mixture after distillation were determined by gas chromatography.

**Table 3**

| Component | Reaction mixture (mol%) | Mixture after distillation (mol%) |
|---|---|---|
| C₈F₁₇CH=CH₂ | 7.04 | 0 |
| C₈F₁₇CH₂CH₂OH | 1.79 | 0.32 |
| C₈F₁₇CH₂CH₂OCOCH=CH₂ | 88.79 | 97.18 |

As shown in Table 3, a highly-pure mixture of the fluorine-containing acrylic esters with less impurities was obtained also in Example 3, similarly to Examples 1 and 2. Specifically, the mixture contained, as the impurity, C₈F₁₇CH₂CH₂OH (n=7) in an amount of 0.32 mol%.

### (Comparative Example 1)

The mixture of the fluorine-containing acrylic esters was obtained similarly to Example 1, and then the distillation was carried out similarly to Example 1. However, the distillation was carried out under the nitrogen atmosphere without using the inhibitor of polymerization and introducing oxygen into the distillation column. As a result, the esters were polymerized in the distillation column, and the intended mixture with a less impurity content was not able to be obtained well. It is considered that this was related also to the fact that the still temperature was made high in order that the ester with a large "n" value (n=7) was intended to be obtained by the distillation.

### (Comparative Example 2)

The ethylene adducts of the fluoroalkyl iodides, which adduct was represented by C₈F₁₇CH₂CH₂I (n=7) was obtained and then this mixture was reacted with the acid ester compound to give a mixture containing the fluorine-containing acrylic esters represented by the formula (1). Specifically, 1533g (2.67 mol) of the ethylene adduct of the fluoroiodide and 320g (2.90 mol) of acrylate potassium, 680mL of tert-butyl alcohol, 1.8g of hydroquinone and 0.32g of hydroquinone monomethylether as the inhibitors of polymerization were firstly charged into an autoclave with a volume of 3L and heated to 180°C to 190°C and reacted for 6 hours. After the reaction, a reaction mixture was cooled. Next, KI as a byproduct was removed by filtration. Thereafter, a filtrate was subjected to distillation to remove tert-butyl alcohol and then 1360g of a reaction mixture having a composition shown in Table 1 was obtained. 500g of this reaction mixture was measured and charged into a still and subjected to distillation at a pressure in a distillation column of 0.9kPa and a still temperature of 160°C, introducing air into the still at a flow rate of 20 ml/min such that the oxygen concentration in the distillation column was 1 mol% relative to the total moles of gas-phase components in the distillation column. Further, the continuous distillation was carried out using an oldershaw-type distillation column with 20 plates which is made of iron as a whole (including the internal structure). As a result of the distillation, 412g of C₈F₁₇CH₂CH₂OCOCH=CH₂ having a composition as shown in Table 1 was obtained as a distillate liquid (a recovery rate based on the fluorine-containing acrylic esters was 89%). In Table 1, the compositions of the reaction mixture and the mixture after distillation were determined by gas chromatography.

**Table 4**

| Component | Reaction mixture (mol%) | Mixture after distillation (mol%) |
|---|---|---|
| C₈F₁₇CH=CH₂ | 7.04 | ND |
| C₈F₁₇CH₂CH₂OH | 1.79 | ND |
| C₈F₁₇CH₂CH₂OCOCH=CH₂ | 88.79 | 97.44 |

Comparative Example 2 is an example wherein the internal structure of the distillation column was made of iron having a low electrode potential. As shown in Table 4, a highly-pure mixture of the fluorine-containing acrylic esters with less impurities was obtained also in Comparative Example 2, similarly to Examples 1, 2 and 3. However, the recovery rate was low and the resultant distillate liquid was colored brownish yellow which could not be used as a product.

As described above, the mixture of the fluorine-containing acrylic esters can be obtained wherein the proportion of the impurities is reduced to a ppm level according to the production method of the present invention. The monomer mixture with less impurities is useful for producing a polymer of high quality. Further, the method of the present invention makes it possible to give the mixture which contains the fluorine-containing acrylic esters with "n" values of 7 and 9 at a high ratio and the fluorine-containing acrylic esters with n≤6 or n≥10 at a low ratio. As described above, the fluorine-containing acrylic esters with n=7 and 9 are monomers which produce the polymer useful as the water- and oil-repellent, and therefore the production method of the present invention contributes to improvement in the quality of the polymer in this point.

### Industrial Applicability

The production method of the present invention makes it possible to obtain the mixture of the fluorine-containing acrylic esters represented by the formula CF₃(CF₂)ₙCH₂CH₂OCOCR¹=CH₂ which mixture contains a high proportion of the esters and a low proportion of the other compounds at a high recovery rate. Therefore, the mixture of the fluorine-containing acrylic esters obtained by this production method is suitable for being used as the monomer for producing various polymers by selecting the "n" value. Particularly, when seven and/or nine is selected as the "n" value, the monomer can be used as the monomer for producing the polymer which is useful as the water- and oil-repellent.

## Claims

1. A method for producing a mixture of fluorine-containing acrylic esters each of which esters is represented by the formula (1):
CF₃(CF₂)ₙCH₂CH₂OCOCR¹=CH₂ (1)
wherein R¹ is a hydrogen atom, a methyl group or a halogen atom and "n" is an integer of at least zero, which comprises:
(a) a step of obtaining a mixture containing a mixture of fluorine-containing acrylic esters each of which esters is represented by the formula (1); and
(b) a step of reducing, in the mixture obtained in the step (a), a proportion of a mixture of fluorine-containing olefins each of which is represented by a formula (2) and/or a mixture of fluoroalkyl alcohols each of which is represented by a formula (3) by subjecting the mixture obtained in the step (a) to distillation:
CF₃(CF₂)ₙCH=CH₂ (2)
wherein "n" is an integer of at least 0,
CF₃(CF₂)ₙCH₂CH₂OH (3)
wherein "n" is an integer of at least 0,
wherein the distillation is carried out using a distillation column equipped with a packing and/or an internal structure made of a material which does not contain a metal having a standard electrode potential of - 0.3V or less and introducing oxygen into the distillation column.

2. The method according to claim 1, wherein the packing and/or the internal structure is made of a resin, a metal oxide, and/or a metal having the standard electrode potential of larger than -0.3V.

3. The method according to claim 1, wherein the distillation is carried out with an oxygen concentration in the distillation column of from 0.1 mol% to 10 mol% based on the total moles of gas-phase components in the distillation column.

4. The method according to claim 1, wherein the step (a) is carried out so that the mixture is obtained which contains the fluorine-containing acrylic esters represented by the formula (1) with n=7 and n=9 in an amount of at least 80 mol% in total, and the distillation in the step (b) is carried out so that a proportion of the fluorine-containing olefins represented by the formula (2) with "n" of at most 9 is substantially 0 mol% and a proportion of the fluorine-containing olefins with "n" of at least 10 is from 0 mol% to 0.05 mol% based on the total moles of fluorine-containing compounds obtained as a distillate.

5. The method according to claim 1, wherein the step (a) is carried out so that the mixture is obtained which contains the fluorine-containing acrylic esters represented by the formula (1) with n=7 and n=9 in an amount of at least 80 mol% in total, and the distillation in the step (b) is carried out so that a proportion of the fluorine-containing alcohols represented by the formula (3) with "n" of at most 8 is substantially 0 mol%, a proportion of the fluorine-containing alcohols with "n" of 9 is from 0 mol% to 0.05 mol% and a proportion of the fluorine-containing alcohols with "n" of at least 10 is from 0 mol% to 0.1 mol% based on the total moles of fluorine-containing compounds obtained as a distillate.

6. The method according to claim 4, wherein the distillation is carried out under conditions wherein a bottom temperature is from 60°C to 160°C, a pressure in the distillation column is from 0.5kPa to 5kPa and a theoretical plate number is from 10 to 35.

7. The method according to claim 5, wherein the distillation is carried out under conditions wherein a bottom temperature is from 60°C to 160°C, a pressure in the distillation column is from 0.5kPa to 5kPa and a theoretical plate number is from 10 to 35.

8. A method for producing a polymer of fluorine-containing acrylic esters each of which is represented by a formula (1):
CF₃(CF₂)ₙCH₂CH₂OCOCR¹=CH₂ (1)
wherein R¹ is a hydrogen atom, a methyl group or a halogen atom and "n" is an integer of at least zero, which comprises:
(a) a step of obtaining a mixture containing a mixture of fluorine-containing acrylic esters each of which esters is represented by the above formula (1); and
(b) a step of reducing, in the mixture obtained in the step (a), a proportion of a mixture of fluorine-containing olefins each of which is represented by a formula (2) and/or a mixture of fluoroalkyl alcohols each of which is represented by a formula (3) by subjecting the mixture obtained in the step (a) to distillation:
CF₃(CF₂)ₙCH=CH₂ (2)
wherein "n" is an integer of at least 0,
CF₃(CF₂)ₙCH₂CH₂OH (3)
wherein "n" is an integer of at least 0,
wherein the distillation is carried out using a distillation column equipped with a packing and/or an internal structure made of a material which does not contain a metal having an electrode potential of -0.3V or less and introducing oxygen into the distillation column; and
(c) a step of polymerizing a mixture obtained in the step (b).

9. A fluorine-containing acrylate polymer produced by the method according to claim 8.

10. A method for purifying fluorine-containing acrylic esters which comprises a step of subjecting a mixture to distillation which mixture contains a fluorine-containing acrylic esters represented by a formula (1), fluorine-containing olefins represented by a formula (2), and fluorine-containing alcohols represented by a formula (3):
CF₃(CF₂)ₙCH₂CH₂OCOCR¹=CH₂ (1)
wherein R¹ is a hydrogen atom, a methyl group or a halogen atom and "n" is an integer of at least zero,
CF₃(CF₂)ₙCH=CH₂ (2)
wherein "n" is an integer of at least 0,
CF₃(CF₂)ₙCH₂CH₂OH (3)
wherein "n" is an integer of at least 0,
in which distillation a distillation column is used which is provided with a packing and/or an internal structure made of a material which does not contain a metal having an standard electrode potential of -0.3V or less and oxygen is introduced, so that a proportion of the fluorine-containing olefins represented by the formula (2) and the fluorine-containing alcohols represented by the formula (3) in the mixture are reduced.
